# EUROPÄISCHE PATENTSCHRIFT

(11) **EP 2 998 398 B1**
(45) Veröffentlichungstag und Bekanntmachung des Hinweises auf die Patenterteilung: **03.01.2018**
(21) Anmeldenummer: 15186025.1
(22) Anmeldetag: 20.11.2009
(51) Int. Cl.: C12N 15/10

(54) **AUTOMATISIERTES SYSTEM ZUR AUFREINIGUNG VON NUKLEINSÄUREN, INSBESONDERE AUS FIXIERTEM GEWEBE**
AUTOMATED SYSTEM FOR PURIFYING NUCLEIC ACIDS, PARTICULARLY FROM FIXED TISSUE
SYSTEME AUTOMATISE DESTINE AU NETTOYAGE D'ACIDES NUCLEIQUES, EN PARTICULIER PROVENANT DE TISSUS FIXES

(30) Priorität: 12.12.2008 DE 102008061714
(43) Veröffentlichungstag der Anmeldung: 23.03.2016
(62) Teilanmeldung aus: 09795728.6
(73) Patentinhaber: Siemens Healthcare Diagnostics Inc., Deerfield, IL 60015-0778 (US)
(72) Erfinder: Euting, Heike, 46399 Bocholt (DE); Henning, Guido, 50859 Köln (DE); Izmailov, Alexandre, Toronto, Ontario M8X 1H4 (CA)
(74) Vertreter: Plate, Jürgen

(56) Entgegenhaltungen:
- A Gleizes ET AL: "Automation of the INVITROGEN ChargeSwitch Forensic DNA Purifi cation kit for Genotyping from forensic samples", , 3. Juli 2006 (2006-07-03), Seiten 1-2, XP055245269, Gefunden im Internet: URL:http://tools.thermofisher.com/content/ sfs/brochures/Hamilton CST Forensic.pdf [gefunden am 2016-01-27]
- Ambion: "High Throughput RNA Recovery from Mammalian, Plant, and Viral Samples", Ambion TechNotes Newsletter, Bd. 13, Nr. 1 Januar 2006 (2006-01), Seiten 24-25, XP002570157, Gefunden im Internet: URL:http://www.ambion.com/techlib/tn/131/A mbionTechNotes13_1.pdf [gefunden am 2010-02-23]
- FANG X ET AL: "High-Throughput Sample Preparation from Whole Blood for Gene Expression Analysis", JOURNAL OF THE ASSOCIATION FOR LABORATORY AUTOMATION, ELSEVIER, Bd. 11, Nr. 6, 1. Dezember 2006 (2006-12-01), Seiten 381-386, XP024969912, ISSN: 1535-5535 [gefunden am 2006-12-01]
- CLAIRE GAILLARD ET AL: "Avoiding adsorption of DNA to polypropylene tubes and denaturation of short DNA fragments.", TECHNICAL TIPS ONLINE, Bd. 3, Nr. 1, 1. Januar 1998 (1998-01-01), Seiten 63-65, XP055245339, United Kingdom ISSN: 1366-2120, DOI: 10.1016/S1366-2120(08)70101-6
- SIEMENS: "True molecular testing versatility to meet your laboratory needs. The VERSANT kPCR Molecular System", 20080101 , 1. Januar 2008 (2008-01-01), Seite 4pp, XP007911896, Gefunden im Internet: URL:https://www.medical.siemens.com/siemen s/en_GLOBAL/gg_diag_FBAs/files/molecular_p df/kPCR_Brochure.pdf [gefunden am 2010-02-24]
- Anonymous: "Ringmagnet Ø 20,0 x 6,4 x 5,0 mm N35H Nickel m. Senkung Süd", , 1. Januar 2016 (2016-01-01), XP055245350, Gefunden im Internet: URL:https://www.magnet-shop.net/detail/ind ex/sArticle/783?gclid=CL-L7Z-OysoCFcWVGwod zp8BBA [gefunden am 2016-01-27]
- Zechbauer U.: "How to Fingerprint a Tumor", Pictures of the Future, November 2008 (2008-11), XP007911894, Gefunden im Internet: URL:http://www.siemens.com/innovation/pool /en/publikationen/publications_pof/pof_fal l_2008/early_detection/cancer_prognosis/po f208_med_cancerprog_pdf_en.pdf [gefunden am 2010-02-24]
- Ambion - The RNA Company: "96 well Magnetic-Ring Stand", , 18. Juni 2008 (2008-06-18), XP002570156, Gefunden im Internet: URL:http://www.ambion.com/techlib/spec/sp_ 10050.pdf [gefunden am 2010-02-22]
- "AGENCOURT FORMAPURE KIT NUCLEIC ACID ISOLATION FROM FORMALIN-FIXED, PARAFFIN- EMBEDDED TISSUE", , 1. Januar 2008 (2008-01-01), Seiten 1-13, XP55088169, Gefunden im Internet: URL:https://www.beckmancoulter.com/wsrport al/bibliography?docname=Protocol 000385v005.pdf [gefunden am 2013-11-13]

## Beschreibung

Die Erfindung betrifft ein automatisiertes System zur Aufreinigung von Nukleinsäuren, das eine Probengefäßhalterung für die Aufreinigung einer biologischen Probe umfaßt.

In jüngster Zeit gewinnt die Molekulare Diagnostik zunehmend an Bedeutung. Sie hat Eingang gefunden in die klinische Diagnostik von Erkrankungen (u. a. Nachweis von Infektionserregern, Nachweis von Mutationen des Genoms, Entdeckung von zirkulierenden Tumorzellen und Identifizierung von Risikofaktoren für die Prädisposition einer Erkrankung). Aber auch in der Veterinärmedizin, der Umweltanalytik und Nahrungsmitteltestung finden Methoden der molekularen Diagnostik mittlerweile ihre Anwendung. Ein weiteres Anwendungsgebiet stellen Untersuchungen an pathologischen/zytologischen Instituten oder im Rahmen forensischer Fragestellungen dar. Aber auch im Rahmen der Gesundheitsvorsorge (z.B. Untersuchungen von Blutkonserven auf Infektionserreger-Freiheit) wird die Gen-Diagnostik mittlerweile eingesetzt und der Gesetzgeber plant, solche Tests per Gesetz in Zukunft anzuordnen. Methoden, die auch bei der klinischen Molekularen Diagnostik zum Einsatz kommen (wie z.B. Hybridisierungs- oder Amplifikationstechniken wie die PCR (Polymerase Chain Reaction), TMA (Transcription mediated amplification), LCR (Ligase Chain Reaction), bDNA (branched DNA) oder NASBA (Nucleic Acid Sequence Based Amplification)-Technologie) gehören auch bei wissenschaftlichen Grundlagenarbeiten zu den Routineverfahren.

Insbesondere eröffnet die Nukleinsäureanalytik durch die Bestimmung der Genexpression in Geweben vielversprechende neue Möglichkeiten in der Erforschung und Diagnostik von Tumorerkrankungen. So haben beispielsweise die sogenannten Mikroarray-Systeme die Möglichkeit eröffnet, in einem einzigen Ansatz die Expression von hunderten oder sogar von tausenden von Genen zu bestimmen. Dabei wird das Probenmaterial, aufgereinigte Nukleinsäuren, z.B. RNA oder cDNA, auf einen Chip aufgebracht, welcher entsprechende Fänger-Oligonukleotide aufweist, so dass die Nukleinsäuren in der Probe durch Hybridisieren nachgewiesen werden können. Daneben existieren auch weit verbreitet andere Verfahren zum Nachweis von Nukleinsäuren in einer Probe, z.B. Amplifikationsverfahren wie die Polymerase Kettenreaktion (PCR).

Ein grundsätzliches Problem in der Nukleinsäureanalytik ist die Probenaufbereitung. Die zu untersuchende Probe enthält meist Zellen oder Gewebe mit störenden, teilweise unlöslichen Bestandteilen (sog. Debris), welche die nachfolgende Isolierung und Analyse stören können. Insbesondere bei der Nukleinsäureisolation von Stuhl/Faeces, Blut, Warzen, verkalkten Strukturen (Knochen) oder auch stark nekrotischem Gewebeproben treten solche unlösliche Bestandteile auf. Debris kann im weitesten Sinne aber auch lösliche Komponenten beinhalten, z.B. freigesetztes Hämoglobin aus Erythrozyten, welches in extremem Überschuss vorhanden ist und während der Isolation von den Nukleinsäuren abgetrennt werden soll.

Dieses Problem ist insbesondere in der Tumordiagnostik gravierend, weil hier als Probenmaterial häufig formalinfixierte Paraffinschnitte (formalin-fixed paraffin-embedded, FFPE-Schnitte) verwendet werden. Bei der Probenentnahme am Patienten, z.B. bei Biopsieentnahme oder intraoperativer Probenentnahme von Tumormaterial, wird Gewebematerial mit Formalin fixiert und in Paraffin eingebettet, um das Probenmaterial haltbar zu machen. Durch die Fixantien kommt es während der Inkubation - aber auch noch über Jahre hinaus innerhalb des Gewebeblockes - zu einer extremen Kreuzvernetzung von Biomolekülen (Nukleinsäuren mit Proteinen sowie jeweils Proteine oder Nukleinsäuren untereinander). Diese vernetzten Strukturen innerhalb und außerhalb von Zellen tragen zur Entstehung von unlöslichem bzw. nicht oder schwer lysierbarem Debris bei. Von den im Paraffin eingebetteten Proben werden üblicherweise Schnitte zur Begutachtung durch Pathologen angefertigt, diese Schnitte können jedoch auch als Ausgangsmaterial in der Nukleinsäureanalytik dienen. Dabei muss bei der Aufreinigung der Nukleinsäuren nach der Lyse sowohl zelluläres Debris als auch das Paraffin entfernt werden.

Darüber hinaus tritt dieses Problem mit störenden, teilweise unlöslichen Bestandteilen (Debris) auch bei der Aufreinigung von Nukleinsäuren aus Stuhlproben (Faeces, Kot) auf. Stuhlproben bestehen zum einen aus den unverdaulichen Anteilen der Nahrung (Ballaststoffe) sowie unverdauten Resten wie Fett, Stärke, Bindegewebs- und Muskelfasern und Wasser, die nicht in den oberen Dickdarmabschnitten resorbiert wurden. An körpereigenen Substanzen sind enthalten: abgestoßene Darmzellen, Rückstände von Verdauungsenzymen und Schleim. Des Weiteren werden geringe Mengen der Gallensäuren selbst, sowie des zum Schutz der Darmschleimhaut ebenfalls von der Galle ausgeschiedenen Lecithins und anderer Phospholipide zusammen mit dem Kot ausgeschieden.

Um Kosten zu senken und die Bearbeitungszeit vom Probeneingang bis zur Bestimmung des Analyseergebnisses so kurz wie möglich zu halten, ist es ein vordringliches Ziel, Verfahren zur Aufreinigung von Nukleinsäuren so effizient wie möglich zu machen und soweit wie möglich automatisiert durchzuführen. Dies gilt insbesondere in der Diagnostik. Gut geeignet zur Automatisierung sind solche Verfahren, welche in möglichst wenig verschiedenen Reaktionsgefäßen durchgeführt werden können und in standardisierten Formaten (z.B. 96-Loch Plattenformat) durchgeführt werden können, weil bei diesem Verfahren effiziente Pipetierroboter eingesetzt werden können. Daher besteht im Stand der Technik das Bedürfnis nach einer einfachen, effizienten und möglichst automatisierbaren Probenaufbereitung.

Übliche Verfahren zur Nukleinsäureaufreinigung umfassen die Probenlyse unter chaotropen Bedingungen, Aufreinigung durch Extraktion, Ausfällen und Aufreinigen aus der flüssigen Phase, z.B. die sog. Phenol-Chloroform-Extraktion (siehe Sambrook et al., Molecular cloning - a laboratory manual, 3. Auflage, Cold Spring Harbor Laboratory, Cold Spring Habor Press, 2001, ISBN-13: 978-0879695774), oder sogenannte säulenbasierte Aufreinigungsverfahren wie beispielsweise offenbart in WO 2003040364-A1.

Ein übliches Verfahren zur Isolierung von Nukleinsäuren wurde von Chomczynski beschrieben (US 5,346,994) und umfasst die Aufreinigung von Nukleinsäuren aus Gewebematerial basierend auf einer Trennung aus der flüssigen Phase unter Verwendung von Phenol und der chaotropen Verbindung Guanidinisothiocyanat. Dabei muss die Probe in wässriger Lösung homogenisiert und nach Zugabe von Guanidinisothiocyanat (GTC) und Phenol/ Chloroform zentrifugiert werden. Proteine befinden sich in der organischen Phase, DNA in der Interphase und RNA in der wässrigen Phase. Die RNA kann aus der wässrigen Phase präzipitiert werden. Dieses Verfahren ermöglicht jedoch nicht die zuverlässige Aufreinigung von RNA aus FFPE-Gewebeproben.

Andere bekannte Verfahren zur DNA- oder RNA-Isolierung verwenden typischer Weise chaotrope Salze oder Phenolextraktion.

EP0819696 offenbart ein Verfahren zur Aufreinigung von Nukleinsäuren, das auf der Bindung von Nukleinsäuren an Silika oder andere Siliziumdioxidderivate unter chaotropen Bedingungen basiert. Dabei wird die Probe in einem chaotropen Lysepuffer lysiert und die Nukleinsäuren an eine Silikamatrix gebunden.

Im Stand der Technik bekannte Verfahren zur Aufreinigung von Nukleinsäuren aus Paraffinschnitten erfordern zunächst eine aufwändige Entparaffinisierung, wobei das Paraffin typischerweise durch Xylol entfernt wird, sowie eine aufwändige anschließende Rehydratisierung mit einer Xylol/Ethanol Verdünnungsreihe.

So wird in der WO 200146402 A1 ein Verfahren zur Aufreinigung von RNA aus fixierten Paraffinschnitten beschrieben, bei welchem der Paraffinschnitt zunächst in ein Eppendorf-Reaktionsgefäß eingebracht und mit Xylol entparaffinisiert wird. Anschließend muss der Schnitt mit einer Xylol/Ethanol Verdünnungsreihe rehydratisiert werden. Anschließend wird zur Aufreinigung der RNA die Probe über einen längeren Zeitraum (5 bis 120 min.) in einer chaotropen Lösung erhitzt. Dieses Verfahren ermöglicht zwar eine effektive Entparaffinisierung, ist aber aufwändig und aufgrund mehrerer erforderlicher Zentrifugationsschritte für eine Automatisierung nicht sehr geeignet.

Ferner ist aus der EP1510577 ein Verfahren bekannt, bei welchen Nukleinsäuren unter chaotropen Bedingungen an magnetische Partikel binden und durch Anlegen eine Magnetfeldes von dem Probenüberstand getrennt werden können. Aus der WO1990014891A1 ist eine magnetische Probenhalterung bekannt, die zu diesem Zweck verwendet werden kann. Bei diesen Verfahren findet jedoch keine vorherige Aufreinigung der Probe von zellulärem Debris bzw. eine Entparaffinisierung unter nicht-chaotropen Bedingungen statt. Die Anwesenheit von Debris in der Probe bei der Aufreinigung von Nukleinsäuren wirkt sich jedoch nachteilig aus und ist insbesondere bei automatisierten Verfahren nachteilig, weil das Debris Pipettenspitzen, Absaugleitungen und Ähnliches verstopfen kann, sowie Drucksensoren, die zum Monitoren des Pipettierschrittes verwendet werden, beschädigen kann.

Bekannt ist ferner ein System mit einer Probengefäßhalterung für eine Anzahl von Proben, die mit einem Greifer zu einem Ringmagnet-Adapter transferiert wird (A. Gleizes et al, Automation in the INVITROGEN ChargeSwitch Forensic DANN Purification kit for Genotyping from forensic samples, Hamilton AG, Switzerland). Magnetische Partikel mit daran haftender DNA werden von den Ringmagneten angezogen und an der Wand des Probengefäßes festgehalten, so dass ein flüssiger Überstand abgezogen werden kann.

Ein als "MagMAX-Kit" bezeichnetes System zur Isolierung von RNA mit Hilfe von Magnetpartikeln ist aus dem Artikel High Throughput RNA from Mammalian, Plant and Viral Samples, in Ambion TechNotes Newsletter, Vol. 13, No. 1, offenbart.

Eine Vorrichtung zur Abtrennung von endogenen Ribonucleasen aus Ganzblutproben ist in dem Artikel von X. Fang et al., High-throughput Sample Preparation from Whole Blood for Gene Expression Analysis, in Journal of the Association for the Laboratory Automation, Vol. 11 [2006], No. 6, Seiten 381 - 386, offenbart. Die Vorrichtung umfaßt ein "MagMAX-96"-Kit zur Isolierung von im Blut enthaltener RNA.

Gegenüber dem Stand der Technik besteht daher ein Bedürfnis nach verbesserten Systemen zur Aufreinigung von Nukleinsäuren, und insbesondere nach Systemen, welche für eine Automatisierung geeignet sind.

### Definitionen

Der Ausdruck "biologische Probe" bezeichnet jegliche Probe, welche Zellen oder zelluläres Material enthält, insbesondere Zellen, gefrorene Zellpellets, fixierte Zellen, Faeces/Stuhl, "Buffy Coat" (= weiße Blutkörperchenfraktion des Blutes), Ascites, Abstriche, insbesondere Backen- oder Rachenabstriche, ganz besonders aber Gebärmutterhalsabstriche, Sputum, Organpunktate, Sperma, Gewebeproben, fixierte Gewebeproben, Gewebeschnitte von fixierten oder nicht-fixierten Gewebeproben, insbesondere Gefrierschnitte und Paraffinschnitte, insbesondere formalinfixierte Paraffinschnitte, Tumormaterial, Biopsieproben, Blutproben, insbesondere Gesamtblut oder Blutfraktionen, Zellsuspensionen und im weitesten Sinne sämtliche Proben, welche zelluläre Bestandteile aufweisen, wobei sowohl intakte Zellen als auch Zellbestandteile umfasst sein sollen.

Darüber hinaus umfasst der Ausdruck "biologische Probe" auch andere nukleinsäurehaltigen, biologischen Materialen, wie z.B. Blutserum oder Blutplasma, insbesondere virushaltiges Serum oder Plasma, dabei ganz besonders HIV und HCV infizierte Serumproben, Sekrete, Liquor, Galle, Lymphflüssigkeit, Urin. Ebenso kann es sich um nukleinsäurehaltige Materialien handeln, welche aus biochemischen oder biotechnologischen Prozessen stammen und anschließend aufgereinigt werden sollen.

Der Begriff "zellulär" bezieht sich sowohl auf prokaryotische als auch eukaryotische Zellen.

Der Begriff "Lysieren der Probe" umfasst das Aufbrechen von Zellen oder zellulären Strukturen in der Probe. Er umfasst insbesondere mechanische Lyseverfahren (z.B. Ultraschall), thermische Lyse (z.B. Einfrier-Auftau-Zyklen, erhitzen der Probe) und chemische Lyse (z.B. mit Detergentien). Der Ausdruck "Lysieren der Probe" ist aber nicht auf Zellen beschränkt und kann sich auch auf das Freisetzen von Nukleinsäuren mit den beschriebenen Verfahren aus nicht-zellulären, biologischen Strukturen oder Komplexen beziehen.

Der Ausdruck "Nukleinsäuren" umfasst oligomere und polymere Ribonukleotide bzw. 2'-Desoxyribonukleotide mit einer Kettenlänge von mehr als 10 Monomereinheiten. Die Monomereinheiten in Nukleinsäuren sind über Phosphorsäurediesterbindungen zwischen 3'- und 5'-Hydroxylgruppe benachbarter Monomereinheiten verknüpft und an das 1'-Atom der jeweiligen Kohlenhydratkomponente ist glykosidisch eine heterocyclische Base gebunden. Nukleinsäuren können durch Ausbildung von intermolekularen Wasserstoffbrückenbindungen Doppel- und Dreifachstränge ausbilden. Ebenso sind Protein-/Nukleinsäurekomplexe sowie Nukleinsäuren mit synthetischen Nukleotiden, wie Morpholinos, LNAs oder PNAs, gemeint.

Der Begriff "chaotrope Bedingungen" bezeichnet Lösungsmittelbedingungen bei Anwesenheit von chaotropen Agentien oder Verbindungen. Chaotrope Agentien oder Verbindungen sind Verbindungen, welche die Sekundärstruktur, Tertiärstruktur und Quaternärstruktur von Proteinen, Nukleinsäuren und Protein-Nukleinsäurekomplexen verändert oder aufbricht, während die Primärstruktur intakt bleibt. In Lösung werden unter chaotropen Bedingungen die intramolekularen Wechselwirkungen von biologischen Molekülen, insbesondere Proteinen, Protein-Nukleinsäurekomplexen und Nukleinsäuren aufgebrochen, da chaotrope Verbindungen mit stabilisierenden intramolekularen Wechselwirkungen in biologischen Molekülen, z.B. Wasserstoffbrückenbindungen, van der Wals-Kräfte und hydrophobe Effekte, interferieren. Chaotrope Verbindungen weisen üblicherweise großvolumige Ionen auf, die aufgrund Ihrer Größe mit den intramolekularen Wechselwirkungen interferieren und dadurch die Polarität des Lösungsmittels herabsetzen können. Dadurch werden inter- und intramoleklare Wasserstoffbrücken aufgebrochen. Infolgedessen präzipitieren viele Proteine, die Helixstruktur von zweisträngigen Nukleinsäureabschnitten bleibt jedoch erhalten. Durch Hinzufügen von chaotropen Verbindungen zu Zelllysaten oder Zellsuspensionen lassen sich Proteine ausfällen, während Nukleinsäuren in Lösung bleiben. Unter chaotropen Bedingungen wird die Bindung von Nukleinsäuren an Silizumdioxid-basierte Matrices stark begünstigt. Chaotrope Verbindungen umfassen beispielsweise hochmolekulare Harnstofflösungen (z.B. 6 bis 8 mol/l Harnstoff), Guanidiniumsalzlösungen (z.B. 6 mol/l Guanidiniumchlorid), hochmolekulare Lithiumsalze (z.B. 4,5 mol/l Lithiumperchlorat). Chaotrope Anionen umfassen die Anionen F-, P0₄³⁻, SO₄²⁻, CH₃C00⁻, Cl⁻, und insbesondere Br⁻, I⁻, N0₃⁻, Cl0₄⁻, SCN⁻ und Cl₃CCOO⁻. Chaotrope Kationen umfassen die Kationen Li⁺, Mg²⁺ , Ca²⁺ , Ba²⁺, und insbesondere das Guanidiniumkation, [CH₆N₃]⁺. Zur Nukleinsäureisolierung bevorzugte chaotrope Verbindung sind Guanidiniumisothiocyanat ([CH₆N₃]⁺ SCN⁻) und Guanidiniumchlorid.

Der Begriff "nicht-chaotrope Bedingungen" bezeichnet Lösungsmittelbedingungen in wässriger und/oder alkoholischer Lösung bei Abwesenheit von chaotropen Agenzien.

Der Begriff "Aufreinigen von Nukleinsäuren" beschreibt das unvollständige oder vollständige Entfernen von Nicht-Nukleinsäurebestandteilen aus einer Nukleinsäure enthaltenden Probe. Er ist nicht beschränkt auf das Erreichen eines bestimmten Reinheitsgrades.

Der Begriff "automatisierte Aufreinigung" umfasst Verfahren, die die manuelle Arbeitskraft von menschlichem Personal ganz oder auch nur in Teilschritten ersetzt und insbesondere bei den Schritten der Aufschließung der biologischen Körperprobe mit einem speziellen Puffer, der Zugabe von magnetischen Partikeln oder alternativen Bindungsverfahren, der Inkubation bei einer bestimmten Temperatur, der Entfernung nicht absorbierter Probenbestandteile, den Waschschritten, der Elution gebundener Nukleinsäuren einer Festphasenmatrix, z.B. von Magnetpartikeln bei einer bestimmten Temperatur und dem Abtrennen des Eluats von der Partikelsuspension Anwendung findet.

Der Begriff "Abtrennen" umfasst die soweit wie mögliche Entfernung von allen biologischen oder chemischen Substanzen oder Komponenten, die nicht das eigentliche Ziel der Isolation - also im Wesentlichen keine Nukleinsäuren - sind. Insbesondere dient die Abtrennung dieser Substanzen der Vermeidung von Interferenzen oder Störeinflüssen während der eigentlichen Bindung, Anreicherung, Aufreinigung und anschließenden Detektion von den Zielmolekülen.

Der Begriff "zelluläres Debris" umfasst sämtliche biologischen Komponenten, die nicht primäres Target einer Nukleinsäureisolation sind und durch einen Reinigungs- oder Negativselektionsschritt von den eigentlichen Zielmolekülen getrennt werden sollen. Nach erfolgter Lyse einer zellulären Probe gehören dazu insbesondere in wässriger Lösung unlösliche und schwer lysierbare Zellbestandteile wie z.B. nekrotisierende Gewebebestandteile, Knochen- oder Kalkstrukturen, insbesondere Mikrocalzifizierungen, des weiteren aber auch geplatzte oder morphologische veränderte Erythrocyten, warzen- und papillomartige Gewebestrukturen, sowie auch spezielle Bakterien, welche eine komplexe, schwer lysierbare Zuckerhülle aufweisen (z.B. Mycobakterien). Des Weiteren gehören Proteine, Membranbestandteile, insbesondere durch Fixierung vernetzte Strukturen etc dazu. In Einzelfällen kann es sich aber auch um in Wasser lösliche Komponenten handeln, welche nach den oben beschriebenen Lyseprozessen freigesetzt werden und abgetrennt werden sollen. Ein Beispiel ist das in großen Mengen und molarem Überschuss gegenüber Nukleinsäuren freigesetzte Hämoglobin nach der Lyse (z.B. durch hypotonische Pufferbedingungen) von Erythrozyten, welches vor der weiteren Verarbeitung der Körperprobe abgetrennt werden soll. Darüber hinaus sind mit "zelluläres Debris" insbesondere alle Komponenten im Faeces/Stuhl gemeint, die keine Nukleinsäuren sind. Stuhl besteht zum einen aus den unverdaulichen Anteilen der Nahrung (Ballaststoffe) sowie unverdauten Resten wie Fett, Stärke, Bindegewebs- und Muskelfasern und Wasser, die nicht in den oberen Dickdarmabschnitten resorbiert wurden. An körpereigenen Substanzen sind enthalten: abgestoßene Darmzellen mit Nukleinsäuren, die isoliert werden sollen, Rückstände von Verdauungsenzymen und Schleim. Des Weiteren werden geringe Mengen der Gallensäuren selbst, sowie des zum Schutz der Darmschleimhaut ebenfalls von der Galle ausgeschiedenen Lecithins und anderer Phospholipide zusammen mit dem Kot ausgeschieden.

Der Begriff "magnetische Partikel" umfasst sowohl organische als auch anorganische magnetische Partikel.

Der Begriff "Silika" umfasst Siliziumdioxid und Siliziumdioxidderivate, insbesondere SiO₂ Kristalle und andere Formen von SiO₂, z.B. mit SiO₂ aufgebaute Diatomeen, Zeolithe, amorphes Siliziumdioxid, Glaspulver, Kieselsäure, Wasserglas sowie Aluminiumsilikate und aktivierte Silikate.

Der Begriff "hydrophobe Matrix" bezeichnet eine Festphase, deren Oberfläche mit einem hydrophoben Material aufgebaut ist, insbesondere ein hydrophobes Kunststoffmaterial, z.B. Polyolefinen, wie PP (Polypropylen), PE (Polyethylen), halogenierte Polyolefine, wie z.B. PTFE (Polytetrafluorethylen), und andere. Die Matrix kann in jeder geeigneten Form vorliegen, z.B. in Form von Partikeln, Fasern, flächige Oberflächen usw. Insbesondere kann die Matrix in Form einer Gefäßinnenwand ausgebildet sein.

Der Begriff "Lysepuffersystem" beinhaltet ein Puffersystem, welches mindestens eine Substanz enthält, die den Aufschluss einer Zelle, eines Zellsystems, Zellbestandteilen oder anderen biologischen Komplexen bzw. Strukturen bewirken oder begünstigen kann. Besonders häufig sind die Substanzen ausgewählt aus der Gruppe von Detergenzien (Triton X-100, SDS, oder ähnliches) sowie enzymatische Reagenzien wie insbesondere Proteinase K. Mit umfasst ist die Verwendung von Reagenzien aus der Gruppe von wässrigen, gepufferten oder ungepufferten Lösungen (im einfachsten Fall Wasser). In einem Lysepuffersytem können ein oder mehrere Komponenten aus einer oder beiden Gruppen oder miteinander kombiniert werden. Im Rahmen dieser Erfindung sind Reagenzien, die chaotrope Substanzen enthalten ausdrücklich nicht als Bestandteil des Lysepuffersystems in den ersten Schritten einer Aufreinigung gemeint.

Die weiteren in der vorliegenden Anmeldung verwendeten Begriffe haben die gewöhnliche, dem Fachmann bekannte Bedeutung.

Die Erfindung betrifft ein automatisiertes System umfassend Mittel, die geeignet sind, ein Verfahren zum Aufreinigen von Nukleinsäuren aus einer biologischen Probe mit den Schritten durchzuführen:
a) Aufnehmen der Probe in einem ersten Probengefäß in einer wässrigen Lösung und Lysieren der Probe unter nicht-chaotropen Bedingungen;
b) Suspendieren von ersten magnetischen Partikeln in der Lösung;
c) Einbringen des ersten Probengefäßes in eine Probengefäßhalterung, wobei das Probengefäß in den Ringinnenraum eines mit der Probegefäßhalterung assoziierten Ringmagneten eingebracht wird;
d) Abtrennen der Lösung von den magnetischen Partikeln; und
e) Isolieren der Nukleinsäuren aus der Lösung,
wobei bei Einbringen des ersten Probengefäßes in den Ringinnenraum des mit der Probengefäßhalterung assoziierten Ringmagneten das jeweilige Probengefäß durch Bewegen in Richtung der Ringachse mindestens einmal aus dem Ringraum hinaus und wieder hinein bewegt wird,
wobei das System
- eine Ablage für mindestens ein Probengefäß;
- eine temperierbare Einrichtung mit mindestens einer Aufnahme für das Probengefäß;
- eine Probengefäßhalterung zur Aufnahme mindestens eines Probengefäßes;
- eine Einrichtung zum Transport der Probengefäßhalterung von der Ablage zu der temperierbaren Einrichtung;
- eine Einrichtung zum Transfer von Flüssigkeit aus einem Probengefäß und/oder in ein Probengefäß; und
- eine Steuerung zum Steuern der Transporteinrichtung, der Flüssigkeitstransfereinrichtung und zum Steuern de Temperatur der temperierbaren Einrichtung aufweist und
die Probengefäßhalterung zur Aufnahme des mindestens einen Probengefäßes mindestens einen ringförmigen Magneten aufweist, in dessen Ringinnenraum das Probengefäß aufgenommen werden kann.

Die Erfindung betrifft ferner eine Probengefäßhalterung, die Bestandteil des Systems ist.

Die Erfindung basiert auf der Erkenntnis, dass zelluläres Debris durch die magnetischen Partikel aus der Lösung sehr effektiv entfernt werden kann. Durch Verwendung ergeben sich mehrere Vorteile: Dadurch, dass das Probengefäß im Ringinnenraum des Magneten aufgenommen wird, wird es in der Halterung zusätzlich stabilisiert. Die magnetischen Kräfte wirken gleichmäßig auf die magnetischen Partikel und an der Wand des Probengefäßes bildet sich eine ringförmige, saumartige Ablagerung der magnetischen Partikel und des zellulären Debris. Dabei ist die Ausbuchtung, welche durch eine ringförmige Ablagerung der Partikel bedingt ist, besonders klein und das Debris ist ringförmig verteilt an der Wand des Probengefäßes abgelagert, so dass die flüssige Probe, z.B. durch Verwendung einer Pipettiervorrichtung effektiv abgezogen werden kann, während das Risiko einer Kontamination durch Berühren der Pipettenspitze mit der Ablagerung minimiert wird. Insbesondere die Ausbildung von igel- bzw. stachelförmig ausstrahlenden Magnetpartikelstrukturen mit den Ablagerungen - wie Sie insbesondere bei Stabmagneten mit einseitig ausgerichtetem Magnetfeld beobachtet wird - wird vermieden. Daher ist dieses Verfahren insbesondere für automatisierte Verfahrensabläufe geeignet.

Insbesondere bietet die Verwendung eines Ringmagneten die folgenden weiteren Vorteile:
Ringmagneten mit einer Feldausrichtung, die parallel zur Symmetrieachse des Rings ist, bewirken die kompakteste Partikelablagerung ohne Ausbildung fadenartiger Strukturen entlang der Feldlinien, wie sie bei Stabmagneten beobachtet wird (sog. "Igelstrukturen).

Ringmagnete minimieren etwaige Fehlpositionierungen der Probengefäße in automatisierten Pipettiersystemen (sog. Pipettierrobotern) und somit die Fehlausrichtung von automatisierten Pipettiervorrichtungen.

Ringmagnete minimieren das Risiko von Partikelverlust oder Kontaminationen in Pipettierrobotern.

Die Erfindung basiert ebenso auf der Erkenntnis, dass die Probengefässhalterung mit dem Ringmagneten in der Lage ist verschiedene funktionelle Schritte einer automatisierten Aufreinigung in einer Materialeinheit zu verbinden; a) die Probenidentifizierung durch Einlesen von Barcodes auf dem Probengefäss in der Probengefässhalterung; b)die Zugabe von Reagentien, wie z.B Lysepuffer und /oder Proteinaase K, sowie Magnetpartikeln c) Nach erfolgter Lyse und Magnetpartikelzugabe die Abtrennung von Debris im Probengefäss durch Magnetisierung in dem Ringmagneten in der Probengefässhalterung. Dabei zeichnet sich das Verfahren insbesondere dadurch aus, dass bei keinem dieser Schritte a-c eine Aspiration von Flüssigkeiten durch Roboterspitzen erfolgen muss. Eine Aspiration und damit verbundener Transfer von Flüssigkeiten in sekundäre Probengefässe erfolgt erst nach der Abtrennung von Debris bzw. störenden Komponenten durch die Magnetpartikel unter nicht-chaotropen Bedingungen.

Die magnetischen Partikel haben gemäß einem Aspekt der Erfindung eine mittlere Größe von <50 pm, bevorzugt <10 pm, ganz bevorzugt <0,5 pm, nicht ausschließend < 0.1 pm, wobei die Größe durch Transmissionselektronmikroskopieverfahren bestimmt wird.

Gemäß einem Aspekt der Erfindung weisen diese Partikel eine siliziumhaltige Beschichtung, insbesondere eine Siliziumdioxid aufweisende Beschichtung auf. Derartige magnetische Partikel sind beispielsweise bekannt aus EP 1468430.

Die Magnetpartikel weisen bevorzugt eine Silikabeschichtung auf, sind also SiO₂ umhüllte magnetische Partikel. Der Ausdruck "SiO₂-umhüllte magnetische Partikel" umfasst Magnetit-Kerne, die aus mindestens 90 Gewichtsprozent Fe₃O₄ bestehen und deren Oberfläche mit Silikat beschichtet ist.

Die magnetischen Partikel sind suspendierbare Partikel, welche durch Anwenden eines externen Magnetfeldes in dem Magnetfeld immobilisiert werden können.

Das Abtrennen der magnetischen Partikel von der Lösung erfolgt nach Immobilisierung der Partikel durch das Magnetfeld, die Lösung kann dann auf jede geeignete Weise von den Partikeln getrennt werden, z.B. durch Abkippen, Absaugen, etc..

Das Isolieren der Nukleinsäuren aus der Probe gemäß Schritt (e) kann mit jedem geeigneten Verfahren erfolgen, z.B. Extraktionsverfahren, säulenbasierte Verfahren, Ausfällen etc.. Der Schritt (e) des Isolierens der Nukleinsäuren ist auf keinen bestimmten Reinheitsgrad der isolierten Nukleinsäuren beschränkt.

In Schritt (a) wird die Probe in einer wässrigen Lösung aufgenommen. Dies kann durch Mischen, Suspendieren, Emulgieren oder Auflösen geschehen. Die Probe kann vor oder nach dem Aufnehmen in wässriger Lösung mechanisch verkleinert werden, beispielsweise durch mechanische Einwirkung (z.B. zerschneiden, rühren), durch Hitzeeinwirkung, durch Ultraschallbehandlung und ähnliche Verfahren. Es ist aber auch möglich, die intakte Gewebeprobe, z.B. einen Gewebeschnitt, direkt in wässriger Lösung zu suspendieren.

Gemäß einem Aspekt der Erfindung ist die Probe eine Blutprobe. Bei der Komplettlyse von Blut wird in großen Mengen und molarem Überschuss gegenüber den Nukleinsäuren Hämoglobin und auch Erythrozyten- sowie Leukozytenmembranbestandteile freigesetzt, welche unter Schritt d)abgetrennt werden können. Der Schritt e) dient dann zur gezielten Aufreinigung der Nukleinsäuren aus der wässrigen Restphase.

Alternativ kann man wie bereits technisch beschrieben selektive Lyse von Erythrozyten durchführen unter z.B. hypotonischen Pufferbedingungen. Dabei werden Hämoglobin und Erythrozytenmembranen in großen Mengen freigesetzt, welche unter Schritt d) abgetrennt werden können. In diesem Falle würde in Schritt e)bei Zugabe eines chaotropen Puffers und evtl. Proteinase K die noch notwendige Leukozytenlyse mit Freisetzung der Nukleinsäuren erfolgen. Diese Verfahrensweisen ermöglichen eine einfache und vollständige Automatisierung der Extraktion von Nukleinsäuren aus Blut, insbesondere Leukozyten, wobei aufwendige Verfahrensschritte wie Zentrifugation und Pelletierung der Leukozyten und Verwerfung des Überstandes vermieden werden können.

Gemäß einem weiteren Aspekt der Erfindung ist die Probe eine Stuhl-/Faecesprobe. Bei der Lyse von Stuhl werden Nukleinsäuren aus gesunden oder krankhaft veränderten, ausgeschiedenen Darmepithelzellen freigesetzt in eine komplexe Matrix aus Zelltrümmern, Ballaststoffen, unverdauten Nahrungsresten wie Fett, Stärke, Bindegewebs- und Muskelfasern, Rückstände von Verdauungsenzymen und Schleim, Gallensäuren, Lecithine und andereren Phospholipiden.

Alle Komponenten der Probe, die keine Nukleinsäuren sind, können unter Schritt d) ganz oder teilweise abgetrennt werden. Der Schritt e) dient dann zur gezielten Aufreinigung der Nukleinsäuren aus der wässrigen Restphase.

Gemäß einem Aspekt der Erfindung ist die biologische Probe eine in Paraffin eingebettete Probe, insbesondere ein Paraffinschnitt, und/oder eine fixierte Probe, insbesondere ein formalinfixierter Paraffinschnitt.

Das Verfahren eignet sich besonders für die Aufarbeitung fixierter Proben, da fixierte Proben z.B. aufgrund von Protein- und Nukleinsäurevernetzung besonders viel Debris aufweisen.

Gemäß einem bevorzugten Aspekt der Erfindung wird die Lösung vor dem Schritt (d) auf mindestens 50°C, bevorzugt 50 - 95 °C, bevorzugt mindestens 60 °C, stärker bevorzugt 60 - 80°C, erhitzt. Dieses Erhitzen ermöglicht vorteilhaft eine bessere Suspension und eine verbesserte Lyse der biologischen Probe in der wässrigen Lösung. Zur effektiveren Lyse ist es bevorzugt, eine Proteinase zuzugeben, insbesondere Proteinase K.

Gemäß einem Aspekt der Erfindung wird vor dem Schritt (d) die Probe wiederum auf unterhalb 50°C abgekühlt. Bei Vorhandensein von Paraffin in der Probe hat das Abkühlen auf unterhalb 50°C den zusätzlichen Vorteil, dass sich das Paraffin wieder verfestigt, z.B. in Form eines Paraffinringes an der Gefäßwand. Die Probe bzw. das Lysat kann dann beispielsweise mit einer Pipettenspitze sehr einfach und akkurat ohne Verstopfungsprobleme abgesaugt werden, wobei das Paraffin in Form des beschriebenen Paraffinringes im Reaktionsgefäß zurückbleibt.

Gemäß einem weiteren Aspekt der Erfindung wird die Probe bzw. das Lysat mit einer hydrophoben Matrix in Kontakt gebracht, in dem sie z.B. in einem Gefäß aus hydrophobem Kunststoffmaterial aufgenommen wird. Dies ist besonders bei der Aufarbeitung von paraffinhaltigen Proben bevorzugt. Als hydrophobe Matrix geeignet sind hierzu beispielsweise die bekannten Reaktionsgefäße von Eppendorf oder Sarstedt, welche aus Polyolefinen (z.B. Polypropylen und Polyethylen) bestehen. Es ist besonders bevorzugt, Paraffin enthaltende Proben in Kontakt mit einer hydrophoben Matrix vor Schritt (d) auf über 50°C zu erwärmen, weil dadurch das Paraffin schmilzt und sich bei Abkühlung vorteilhaft als Ring an der Flüssigkeitsoberfläche an der Matrix, z.B. bei einem Kunststoffreaktionsgefäß am Gefäßrand absetzt. Dies geschieht durch Absorptionsvorgänge des verflüssigten Paraffins an der hydrophoben Matrix. In Folgeschritten kann deswegen vorteilhaft anschließend die flüssige Probe akkurat ohne Verstopfungen von Pipettenspitzen abgesaugt werden, während der Paraffinring im Reaktionsgefäß zurückbleibt.

Gemäß eines weiteren Aspekts der Erfindung ist das beschriebene Verfahren bzgl. der Aufreinigungseffizienz so gut, dass für die meisten Anwendungen ein einzelner 3 - 20 pm, ganz besonders bevorzugt ein einzelner 10 µm Paraffinschnitt ausreicht, um sehr hohe Ausbeuten von Nukleinsäuren zu erzielen. Dadurch ist die eingesetzte Paraffinmenge unterhalb von kritischen Mengen, welche eine Ausbildung des Ringes verhindern oder stören.

Gemäß einem Aspekt der Erfindung wird die Lösung in Schritt (d) durch Absaugen von den Magnetpartikeln abgetrennt.

Gemäß einem Aspekt der Erfindung umfasst Schritt (e) ferner das Hinzufügen einer chaotropen Verbindung zu der Lösung. Dabei ist die erstmalige oder wiederholte Hinzugabe von Proteinase K (falls in Schritt a) schon Proteinase K verwendet wurde) in Schritt e), d.h. bevor oder nach der Zugabe der chaotropen Lösung nicht ausgeschlossen.

Gemäß einem Aspekt der vorliegenden Erfindung umfasst Schritt (e) ferner das Hinzufügen von unverbrauchten (frischen) magnetischen Partikeln mit siliziumhaltiger Beschichtung zu der Lösung.

Zur Isolation von RNA ist es bevorzugt, eine DNase in biologisch wirksamer Menge zu der Probe hinzuzufügen. Dadurch wird DNA "verdaut" und geht in Lösung, während die unverdaute RNA aus der Lösung isoliert werden kann. Der DNase Verdau kann zu unterschiedliche Zeitpunkten der Extraktion durchgeführt werden, frühestens nach der Lyse und spätestens nach der Elution am Ende der Aufreinigung.

Zur Aufreinigung von DNA ist es bevorzugt, zu der Probe RNase in biologisch wirksamer Menge hinzuzufügen, wodurch RNA verdaut und die intakte DNA aus der Probe isoliert werden kann. Der RNase Verdau kann zu unterschiedliche Zeitpunkten der Extraktion durchgeführt werden, frühestens nach der Lyse und spätestens nach der Elution am Ende der Aufreinigung. Bevorzugt ist allerdings den DNA Nachweis in Gegenwart der mitaufgereinigten RNA zu führen, also unter Weglassen des RNase Schrittes oder unter Verwendung von Pufferbedingungen, welche eine selektive Isolierung von DNA unter Ausschluss der RNA ermöglichen.

Bei Einbringen des Probengefäßes in den Ringinnenraum des mit der Probegefäßhalterung assoziierten Ringmagneten wird das jeweilige Probengefäß durch Bewegen in Richtung der Ringachse mindestens einmal aus dem Ringraum hinaus und wieder hinein bewegt. Dadurch werden die abgelagerten magnetischen Partikel gemeinsam mit dem Debris und mit eventuell vorhandenen Paraffinresten besonders effektiv ringförmig an der Wand der Probengefäßes abgelagert und verteilt, so dass eine etwaige Ausbuchtung der Ablagerung weiter minimiert wird. Im Gegensatz zu konventionellen Stabmagneten mit einem einseitig wirkenden Magnetfeld verhindern die Ringmagnete insbesondere die Ausbildung von speziellen, makroskopisch sichtbaren stachel- oder igelförmigen Magnetpartikelstrukturen.

Bei üblichen Verfahren der Nukleinsäureaufreinigung wird das Probenmaterial in einem chaotropen Puffer aufgenommen bzw. lysiert. Die vorliegende Erfindung basiert auf der überraschenden Erkenntnis, dass eine Nukleinsäureaufreinigung zu verbesserten Ergebnissen führt, wenn vor dem Isolieren der Nukleinsäuren aus der Probe zelluläres Debris unter nicht-chaotropen Bedingungen entfernt wird. Dies kann beispielsweise durch Zentrifugation oder Filtrieren geschehen. Gemäß einer Ausführungsform der Erfindung wird das Debris mit Hilfe von magnetischen Partikeln unter nicht-chaotropen Bedingungen entfernt. Bevorzugt weisen diese Partikel eine siliziumhaltige Beschichtung, insbesondere eine Siliziumdioxid aufweisende Beschichtung auf. Solche Partikel sind bekannt aus EP 1468430, welche durch Bezugnahme hierin inkorporiert wird. Die Herstellung derartiger Partikel wird weiter unten ausführlich beschrieben.

Die Isolierung von Nukleinsäuren aus dem von Debris gereinigten Lysat kann durch bekannte Verfahren erfolgen. Geeignet sind beispielsweise Extraktionsprotokolle, die auf einer Aufreinigung aus chaotropen Lösungen basieren, z.B. durch Fällung der Nukleinsäuren und/oder Absorption an silikahaltige Matrices unter chaotropen Bedingungen. Bei bekannten säulenbasierten Verfahren werden aus dem Lysat die Nukleinsäuren in Gegenwart chaotroper Salze, die in hoher Konzentration zugegen sind, an eine Silika-Membran gebunden und nach einem Reinigungsschritt von der Membran eluiert. Entsprechende Kits sind von der Fa. QIAGEN GmbH, Hilden, Bundesrepublik Deutschland, kommerziell erhältlich.

Gemäß einem bevorzugten Aspekt der Erfindung erfolgt die Isolierung der Nukleinsäuren durch eine erneute Anwendung von (frischen) silikabeschichteten Magnetpartikeln unter chaotropen Bedingungen.

Gemäß einem bevorzugten Aspekt der Erfindung ermöglicht die Abtrennung von Debris bzw. störenden Substanzen auch die Erhöhung der Aufreinigungseffizienz, Reproduzierbarkeit und Robustheit, sowie die Reduzierung von Ausreißern und nicht eindeutigen bzw. unklaren Ergebnissen in der Analyse der Nukleinsäuren ("Flagging" von Ergebnissen, "geflagte" Resultate). Dies geht in der klinischen Diagnostik, wo solche Verfahren neben der Forschung auch eingesetzt werden sollen, einher mit sogenannten Wiederholungs- bzw. Reflextests, die zusätzliche und vermeidbare Kosten bedeuten.

Die Probengefäßhalterung zur Aufnahme des mindestens einen Probengefäßes weist mindestens einen ringförmigen Magneten auf, in dessen Ringinnenraum das Probengefäß aufgenommen werden kann.

Bevorzugt beträgt der Ringinnendurchmesser des ringförmigen Magneten an seiner engsten Stelle 4 bis 50 mm, 4 bis 20 mm, 5 bis 15 mm, bevorzugt 6 bis 12 mm, insbesondere 8 mm.

Der Ringinnenraum weist einen konischen Bereich auf, der sich bevorzugt der Form und Geometrie eines jeweiligen Probengefässes anpasst.

Die Probengefäßhalterung kann eine Mehrzahl von Aufnahmen für Probegefäße aufweisen, z.B. 2 bis 1600, bevorzugt 2 bis 96, bevorzugt 2, 4, 6, 8, oder 12. Diese Aufnahmen können in einer Reihe angeordnete sein oder in einer orthogonalen Matrixanordnung, insbesondere 2x2, 2x3, 4x6, 6x8, 8x12, 16x24 oder 32x48, wie sie in der In-Vitro Diagnostik häufig verwendet werden.

Im Folgenden wird die Erfindung beschrieben anhand von detaillierten Beispielen im Zusammenhang mit den Figuren, in welchen gezeigt ist:
- FIG 1:: eine schematische Darstellung einer Probenhalterung mit befestigtem Stabmagneten mit einseitig wirkendem Magnetfeld gemäß dem Stand der Technik.
- FIG 2:: eine schematische Darstellung in Draufsicht einer Probenhalterung welche im Verfahren verwendet wird.
- FIG 3:: eine schematische Schnittdarstellung einer Probenhalterung welche im Verfahren verwendet wird.
- FIGS 4 und 5:: eine schematische Darstellung der unterschiedlichen Ausprägung der Ablagerung von Partikeln in einer Probenhalterung gemäß dem Stand der Technik (Fig. 4) versus einer Probenhalterung welche im Verfahren verwendet wird (Fig. 5).
- FIG 6:: eine schematische Darstellung einer Weiterbildung des Verfahrens.
- FIG 7:: eine schematische Darstellung des Verfahrens.

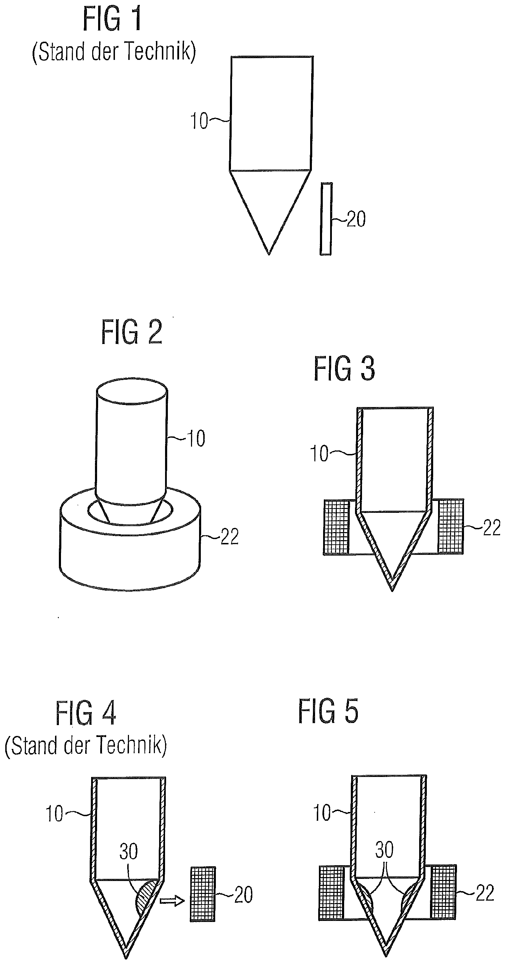

In Figur 1 ist schematisch eine Anordnung aus Probengefäß 10 und Magnet 20 gemäß dem Stand der Technik gezeigt. Eine solche Anordnung ist z.B. aus WO1990014891A1 bekannt.

Figuren 2 und 3 zeigen die erfindungsgemäß verwendete Anordnung aus Probengefäß 10 und ringförmigen Magnet 22. Der Magnet 22 kann ein Ringmagnet sein wie er z.B. von der Firma K&J Magnetics Inc. Jamison PA 18929, USA, in zahlreichen Größen vertrieben wird. Bevorzugt werden starke Neodym-Magnete verwendet, also Magnete, welche Neodymhaltige Legierungen aufweisen, z.B. NdFeB. Eine bei der Verwendung der in der Diagnostik weit verbreiteten Eppendorf-Reaktionsgefäße (Fassungsvolumen/Größe 1,5-2,5 ml) besonders geeignete Größe des Ringmagneten ist ein Aussendurchmesser von 1 bis 2 cm, z.B. 12 mm und ein Innendurchmesser von 5-12 mm, z.B. von 8 mm. Ein 1,5 ml Eppendorf-Reaktionsgefäß hat einen Außendurchmesser im zylindrischen Teil von 11 mm, so dass es bei Verwendung eines Ringmagneten mit Innendurchmesser von 8 mm mit dem konischen Teil des Reaktionsgefäßes in dem Ringinnenraum des Magneten aufgenommen werden und von diesem stabilisiert oder gehalten werden kann. Übliche Probengefäßes haben an der Öffnung einen Kragen, so dass es möglich ist, den Magneten so zu wählen bzw. zu dimensionieren, dass der Innendurchmesser kleiner als der Durchmesser des Kragens, aber größer als der Durchmesser des zylindrischen Bereichs unterhalb des Kragens des Probengefäßes ist. Auf diese Weise kann das Probegefäß vom Ringmagneten gehalten werden.

Der Ringinnenraum kann auch konisch oder teilweise konisch ausgebildet sein um komplementär zu einem konischen Bereich des Probengefäßes dieses passend aufzunehmen.

In Figur 4 ist gezeigt, dass bei Verwendung einer Probenhalterung gemäß dem Stand der Technik eine stark ausgebuchtete Ablagerung 30 der Magnetpartikel entsteht, während bei Verwendung der erfindungsgemäßen Probenhalterung eine ringförmige Ablagerung mit geringer Ausbuchtung entsteht (Figur 5). Ferner lässt sich erkennen, dass sich hierbei die magnetischen Kräfte ausgleichen, während bei der in Fig. 4 dargestellten Situation das Probengefäß in Pfeilrichtung von dem Magneten 20 abgelenkt wird, was insbesondere bei automatisierten Verfahren unerwünscht ist, da das Risiko einer unbeabsichtigten Berührung der Absaugpipette (nicht gezeigt)mit der Ablagerung 30 oder des Probengefäßes 10 steigt. Darüberhinaus werden durch die Verwendung des in Fig. 5 dargestellten Ringmagneten insbesondere die Ausbildung von igel- bzw. stachelförmig ausstrahlenden Magnetpartikelstrukturen - wie sie insbesondere bei Stabmagneten analog zu Fig. 4 mit einseitig ausgerichtetem Magnetfeld beobachtet wird - vermieden.

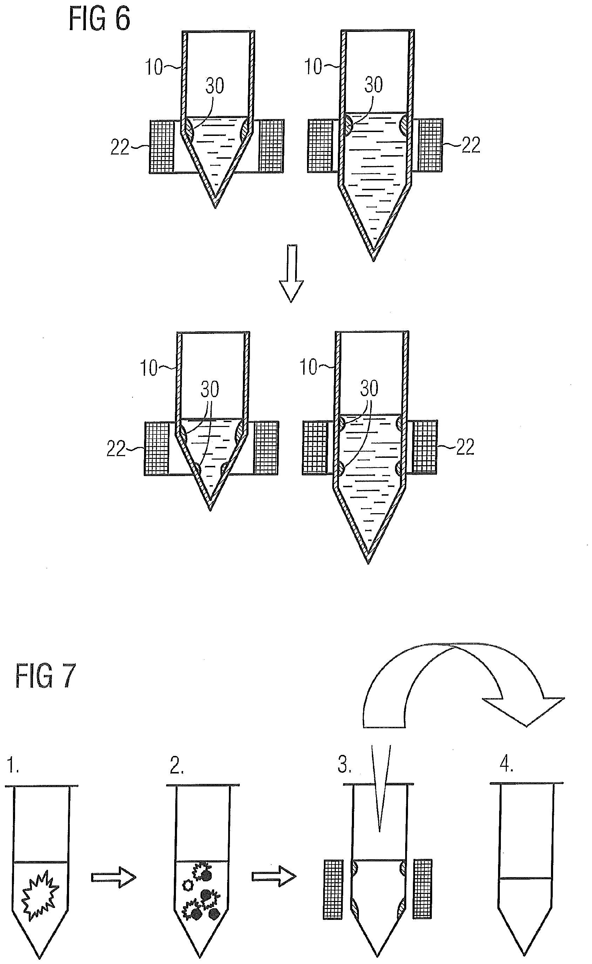

Wie in Fig. 6 gezeigt, kann bei Einbringen des Probengefäßes in den Ringinnenraum des mit der Probegefäßhalterung assoziierten Ringmagneten das Probengefäß 10 durch Bewegen in Richtung der Ringachse mindestens einmal aus dem Ringraum hinaus und wieder hinein bewegt werden. Dadurch werden die abgelagerten magnetischen Partikel 30 gemeinsam mit dem Debris und mit eventuell vorhandenen Paraffinresten besonders effektiv ringförmig an der Wand der Probengefäßes abgelagert und verteilt, so dass eine etwaige Ausbuchtung der Ablagerung weiter minimiert wird. Dies ist insbesondere bei der beschriebenen Aufreinigung von Paraffinschnitten vorteilhaft, weil nun das Paraffin an der Gefäßwand "verschmiert" und verteilt wird und das Risiko einer verstopften Absaugvorrichtung minimiert wird.

Figur 7 stellt das Verfahren schematisch dar. Es kann manuell oder automatisiert ablaufen.

### BEISPIELE:

### Material und Methoden:

### Folgende Materialien und Methoden wurden in allen folgenden Beispielen verwendet

Als Ausgangsmaterial dienen Tumorproben aus einem klinischpathologischen Labor, welche zum Zeitpunkt der Entnahme in Formalin fixiert und anschließend in Paraffin eingebettet wurden. Diese Verfahren zur Fixierung und Einbettung sind dem Fachmann allgemein bekannt und werden hier nicht näher beschrieben. Mit einem Mikrotom werden von der Probe Gewebeschnitte, z.B. mit einer Dicke von 5 bis 10 pm, erhalten und in ein 1,5 ml Probengefäß überführt, z.B. ein 1,5 ml Polypropylen-Probengefäß (wie z.B. das bekannte "Eppendorf-Gefäß"). Alternativ können auch Proben, welche bereits auf einen Objektträger aufgebracht wurden, von diesem mit einer Rasierklinge oder auf andere geeignete Weise (z.B. Entparaffinisierung mit Ethanol/Xylol)abgelöst oder heruntergekratzt und in das Probengefäß überführt werden.

Neben den kommerziell erhältlichen "Versant kPCR Sample Preparation Reagents der Firma Siemens Healthcare Diagnostics GmbH (Erlangen, Deutschland) (bestehend aus einer Proteinase K Lösung, Bindungspuffer (enthält Chaotrop z.B. 59% Guanidinthiocyanat und 10% Octylphenoxypolyethoxyethanol), silicabeschichtete magnetische Partikel, wie z.B. aus EP 1468430 bekannt, Waschpuffer 1 (enthält Chaotrop bzw. 36% Guanidinthiocyanat und 30% Ethanol, Waschpuffer 2 (enthält 80% Ethanol), Waschpuffer 3 (enthält 5-Chlor-2-methyl-4-isothiazolin-3-on und 2-Methly-4-isothiazolin-3-on (3:1)) und Elutionspuffer (enthält Natriumazid) wurden folgende Puffer verwendet:
1. FFPE Lysepuffer
   10 mM Tris-HCl
   0.1 mM EDTA
   2% SDS
   pH 8.0
2. DNA-free DNAse Lösung(Ambion, Cat# A 1906, Ambion, Foster City, CA 94404, USA)

Anstatt der Puffer der kommerziell erhältlichen "Versant kPCR Sample Preparation Reagents" der Firma Siemens Healthcare Diagnostics GmbH können auch andere übliche, dem Fachmann bekannte Pufferzusammensetzungen gewählt werden. Als FFPE Lysepuffer kommen insbesondere detergenzhaltige und/ oder hypotone Puffer in Betracht. Geeignete Waschpuffer sind ebenfalls aus dem Stand der Technik bekannt und kommerziell erhältlich. Als Bindungspuffer für die nachfolgende Isolierung von Nukleinsäuren mit silica-beschichteten Magnetbeads aus dem Lysat kommen chaotrope Pufferzusammensetzungen in Frage, z.B. 4.5 M Guanidinium HCl, 6M Guanidiniumisothiocyanat u. Ä.. Die Anforderungen, die an geeignete Waschpuffer zu stellen sind, bestehen lediglich darin, dass der Puffer gewährleisten muss, dass die Nukleinsäure nicht von der Silica-Matrix abgelöst wird. Allgemein genügt ein hoher Alkoholgehalt und optional leicht alkalischer pH, um die Autoproteolyse der DNA zu verhindern. Auch Waschpuffer, die chaotrope Verbindungen enthalten sind geeignet, solange sie die oben erwähnten Bedingungen erfüllen. Als Elutionspuffer kommen auch dem Fachmann bekannte Pufferzusammensetzungen in Frage, z.B. TE Puffer (10 mM Tris-HCl, 0.1 mM EDTA, pH 8.0).

Es wird darauf hingewiesen, dass in diesem und ähnlichen Aufreinigungsprotokollen die RNA zu 100 bis 500 Basenpaaren langen Fragmenten fragmentieren kann, für Expressionsanalyse mit gängigen Verfahren (RT-PCR, Micro-Array u.ä.) ist jedoch auch fragmentierte RNA gut geeignet.

Die (relative) Quantifizierung RNA-Ausbeute fand mittels der einstufigen kinetischen Real-Time Reverse Transkriptase Polymerase Kettenreaktion (One-Step kRT-PCR) mit Hilfe einer TaqMan Sonde statt. Zur Analyse der RNA Ausbeute wurde der CT-Wert (cycle treshold, d.h. der Wert des Amplifikationszyklusses, welcher als erster einen definierten Schwellwert übersteigt) für die RNA des Referenz- bzw. Housekeeping-Gens RPL37A bestimmt, d.h. die mRNA des humanen Gens für ribosomales Protein L37a, Genbank-Accesion Number NM_000998. Die q RT-PCR wurde unter Verwendung des "SuperScript™ one-step with a Platinum® Taq kits" der Firma Invitrogen, Karlsruhe, Deutschland, und unter Verwendung von Primern und einer Sonde der Firma Eurogentec, Köln, Deutschland durchgeführt.

Zur Durchführung einer kRT-PCR Expressionsanalyse von RPL37A wurde 1 µl aufgereinigte RNA zu 9 µl Mastermix hinzugegeben, bestehend aus 400 nM forward primer, 400 nM reverse primer, 200 nM Taqman-Sonde (FAM/ TAMRA markiert), Reaktionsmix mit jeweils 0,2 mM dNTP und 1,2 mM Magnesiumsulfat sowie 1 µl Platinum® Taq Mix. Die Reaktion wurde auf einem ABI7900 Gerät der Firma Applied Biosystems, Applera Deutschland GmbH, Darmstadt, Deutschland, mit folgendem Temperatur-Profil durchgeführt:
- 30 min. bei 50°C
- 2 min. bei 95°C
- 15 sek. bei 95°C
- 30 sek. bei 60°C, 40 Zyklen

Zur Ermittlung der CT-Werte wurde die Software SDS 2.0 von Applied Biosystems entsprechend der Betriebsanweisung verwendet. Der CT-Wert entspricht der Anzahl von Amplifikationszyklen, ab welcher das Amplifikationssignal eine definierte Schwelle, z.B. die Messschwelle, überschritten hat. Je mehr Nukleinsäure bzw. RNA bzw. DNA in der Probe vorliegt, um so geringer ist dementsprechend der CT Wert. Zum Teil wurden die CT-Werte für RPL37A in den Abbildungen zwecks besserer Darstellung als 40-CT angegeben. Auf diese Weise werden die Werte invertiert und höhere 40-CT Werte entsprechen höheren Expressionswerten von RPL37A. Wenn nicht näher spezifiziert ist der direkt gemessene CT-Wert gemeint.
Beispiel 1: Isolierung von RNA aus einem formalinfixierten Paraffinschnitt mit Entfernung von Debris durch Zugabe von magnetischen Partikeln unter nicht-chaotropen Bedingungen.

Dieses Verfahren entspricht dem in Fig. 7 schematisch dargestellten Verfahren.

RNA aus FFPE-Gewebe Schnitten wurde folgendermaßen manuell aufgereinigt:
- FFPE-Gewebe Schnitt in einem Eppendorf-Probegefäß 1 min bei maximaler Geschwindigkeit zentrifugieren
- Zugabe von 150 µl FFPE Lysepuffer und 50 µl Proteinase K
- 2 h bei 65°C unter Schütteln inkubieren (entspricht Schritt 1. gemäß Fig. 7)
- Zugabe von 50 µl magnetischer Partikel
- Mischen durch 2 min Schütteln (entspricht Schritt 2. gemäß Fig. 7)
- Einbringen des Probengefäßes in den Ringinnenraum des Magneten, evtl. nochmaliges Auf- und Abbewegen des Probengefäßes) (entspricht Schritt 3. gemäß Fig. 7)
- Überstand z.B. durch Pipettieren vorsichtig in ein neues Gefäß überführen (Zell Debris und Paraffin-Reste verbleiben im alten Gefäß) (entspricht Schritt 4. gemäß Fig. 7)

Im folgenden können aus dem Überstand die Nukleinsäuren iosliert werden, z.B. durch:
- Zugabe von 800 µl Bindungspuffer (Chaotrop)
- Zugabe von 50 µl magnetischer Partikel
- Inkubation für 15 min. bei Raumtemperatur unter Schütteln;
- Anlegen eines Magnetfeldes, Absaugen und Verwerfen des Überstands;
- Entfernen des Magnetfeldes. Aufnehmen und Suspendieren der magnetischen Partikel (mit den daran gebundenen Nukleinsäuren) in 850 µl Waschpuffer 1;
- Anlegen des Magnetfeldes, Absaugen und Verwerfen des Überstands;
- Entfernen des Magnetfeldes. Aufnehmen und Suspendieren der magnetischen Partikel (mit den daran gebundenen Nukleinsäuren) in 450 µl Waschpuffer 2;
- Anlegen des Magnetfeldes, Absaugen und Verwerfen des Überstands;
- Entfernen des Magnetfeldes. Aufnehmen und Suspendieren der magnetischen Partikel (mit den daran gebundenen Nukleinsäuren) in 450 µl Waschpuffer 3;
- Wiederholtes Waschen mit Waschpuffer 3;
- Nach Anlegen des Magnetfeldes und Entfernen des Überstandes aufnehmen der Probe in 100 µl Elutionspuffer, Inkubation 10 min. bei 70°C unter Schütteln in einem Thermomixer;
- Anlegen eines Magnetfeldes, Überführen des Eluats in ein frisches Probengefäß.
- Zugabe von 10 µl 10x DNAse Puffer und 1 µl DNAseI
- 30 min bei 37°C inkubieren
Einfrieren der Proben und/ oder weitere Analyse der Eluate.

In Figur 7 ist das Verfahren schematisch dargestellt. Es kann manuell oder automatisiert ablaufen.

Beispiel 2: Automatisierte Aufreinigung von RNA aus formalinfixierten Gewebeschnitten unter Verwendung eines zusätzlichen Bindungsschritts mit magnetischen Partikeln zum Abtrennen von zellulärem Debris unter nicht-chaotropen Bedingungen

RNA aus formalinfixierten Paraffinschnitten wurde aufgereinigt unter Verwendung des folgenden automatisierten Protokolls auf einer Siemens Plattform VERSANT kPCR (Extraktionseinheit). Bis zu 48 Gewebeschnitte können in einem Durchlauf aufgereinigt werden.

### Probenvorbereitung

Gewebeschnitte (5 - 10 pm) wurden durch Zentrifugation bei Raumtemperatur pelletiert und auf Probenträger der Siemens Molekularen Plattform VERSANT kPCR platziert, wo sämtliche Hardwaremodule (Probengefäßhalterung mit Ringmagneten, Heater/Shaker, Magneten etc), Probengefäße, Puffer und Pipettenspitzen an ihren designierten Positionen platziert sind. Start Aufreinigungsprogramm:
- Beladung des Roboters mit einem oder mehreren Probengefässen, die in eine Probengefässhalterung mit Ringmagneten eingebracht werden
- Starten des Aufreinigungsprogramms
- Einziehung der Probengefässhalterungen in den Roboterraum
- Probengefässidentifizierung und Tracking durch Einlesen eines auf dem Probengefäss fixierten Barcodes in der Probengefässhalterung
- Hinzufügen von 150 µl Lysepuffer zu Proben in dem Probengefäß in einer Probengefäßhalterung mit Ringmagneten;
- Hinzufügen von 50 µl Proteinase K-Lösung;
- Übertragen der Probengefäße auf einen Heizschüttler und Inkubation für 2 Stunden bei 65°C unter Schütteln
- Hinzufügen von 50 µl Magnetpartikelsuspension;
- Inkubation 10 min. bei 65°C unter Schütteln;
- Inkubation 5 min. ohne Schütteln;
- Transfer der Probengefäße vom Heizschüttler zurück in die Probengefäßhalterung und Einbringen der Probengefäße in den Ringinnenraum des Magneten, evtl. nochmaliges Auf- und Abbewegen des Probengefäßes) (entspricht Schritt 3. gemäß Fig. 7
- Magnetisieren der Proben 3 min.;
- Transfer des Überstands zu einer Deep-well Probenplatte (DWP);
- Hinzufügen von 600 µl Bindungspuffer (Chaotrop);
- Hinzufügen von 50 µl Magnetpartikelsuspension in die DWP;
- Inkubation für 10 min. bei Raumtemperatur unter Schütteln;
- Transfer der DWP zu Magneten;
- Inkubation 5 min. bei Raumtemperatur im Magnetfeld;
- Absaugen und Verwerfen des Überstands;
- Transfer der DWP von Magnet zum Heizschüttler;
- Hinzufügen von 850 µl Waschpuffer 1;
- 10 sek. Schütteln bei Raumtemperatur;
- Transfer der DWP zum Magneten;
- Magnetisierung 2 min. bei Raumtemperatur;
- Absaugen und Verwerfen des Überstands;
- Transfer der DWP vom Magneten zum Heizschüttler;
- Hinzufügen von 450 µl Waschpuffer 2;
- 10 sek. Schütteln bei Raumtemperatur;
- Transfer der DWP zum Magneten;
- Magnetisierung 2 min. bei Raumtemperatur;
- Absaugen und Verwerfen des Überstands;
- Transfer der DWP von Magnet zum Heizschüttler;
- Hinzufügen von 850 µl Waschpuffer 3;
- 10 sek. Schütteln bei Raumtemperatur;
- Transfer der DWP zum Magneten;
- Magnetisierung 2 min. bei Raumtemperatur;
- Absaugen und Verwerfen des Überstands;
- Hinzufügen von 100 µl Elutionspuffer;
- Transer der DWP von Magnet zum Heizschüttler;
- Inkubation 10 min. bei 70°C unter Schütteln;
- Tranfer der DWP zum Magneten;
- Hinzufügen von 12 µl DNase-Mix (10 µl 10x DNase-Puffer; 2 µl DNase 1);
- Transfer der DWP von Magnet zum Heizschüttler (heruntergekühlt auf 37°C);
- Inkubation 30 min. bei 37°C ohne Schütteln;
- Transfer der DWP zu Magnet;
- Übertragen der DNase-verdauten Proben auf 1,5 ml Probengefäße;

### Ende Aufreinigungsprogramm

- Einfrieren der Proben und/ oder weitere Analyse der RNA Ausbeute.

Es zeigt sich, dass die Aufreinigung mit der erfindungsgemäßen Entfernung von zellulärem Debris zu einer signifikant höheren Ausbeute im Vergleich zu Proben führt, bei denen das zelluläre Debris nicht entfernt wurde. Der Ausbeutevergleich erfolgt über quantitative PCR des housekeeping-Gens RPL37A, wobei als Angabe für die Transkriptmenge 40-CT (CT = cycle threshold, d.h. die Anzahl von Amplifikationszyklen, bei welchen die Messschwelle des Systems überschritten wird). Es ergibt sich dabei eine verbesserte Ausbeute mit einer Differenz von 3 bis 5 CT-Werten von RPL37A. Dies entspricht einer Verbesserung von Faktor 8 bis 32 der Ausbeute der Gesamt-RNA. Dies führt zu der Schlussfolgerung, dass einerseits unlysiertes Gewebe bzw. zelluläres Debris die effiziente und quantitative Aufreinigung von Nukleinsäuren, insbesondere von RNA, beeinträchtigt und vermutlich die Bindung von Nukleinsäuren an silikabeschichtete magnetische Partikel unter chaotropen Bedingungen stört. Es zeigt sich auch, dass sich eine Zentrifugation der lysierten Probe unter nicht-chaotropen Bedingungen zur Entfernung von zellulärem Debris durch einen zusätzlichen Aufreinigungsschritt mit silikabeschichteten magnetischen Partikeln unter nicht-chaotropen Bedingungen substituieren lässt. Dies hat den erheblichen Vorteil, daß das Verfahren damit sehr viel einfacher automatisierbar ist, da keine weiteren Zentrifugationsschritte notwendig sind.

Es zeigt sich ferner, dass der zusätzliche magnetische Aufreinigungsschritt zur Entfernung von Debris unter nicht-chaotropen Bedingungen auch zwischen den aufeinanderfolgenden Schnitten aus einer Gewebeprobe zu besser reproduzierbaren Ergebnissen führt (geringere Varianz innerhalb von unterschiedlichen RNA Präparationen aus dem gleichen Paraffinblock) .

Das VERSANT kPCR System der Firma Siemens, welches einen Pipettierrobotor der Firma Hamilton beinhaltet, ermöglicht die Kontrolle aller Aspirations- und Dispensionsschritte für jeden einzelnen Pipettierschritt. Die Flüssigkeitsbewegungen werden über Drucksensoren, die in den einzelnen Pipettierkanälen enthalten sind, aufgezeichnet. Diese Änderungen der Druckverhältnisse während jedes Pipettierschrittes werden über die Zeit aufgezeichnet (= TADM, Total Aspiration and Dispense Monitoring). Für jeden Pipettierschritt können bestimmte Toleranzbereiche für die Änderung des Druckverhältnisse definiert werden. Sobald das TADM-Profil außerhalb des definierten Bereiches liegt, kann direkt vermerkt werden, dass der Pipettierschritt bei einer Probe nicht ordnungsgemäß durchgeführt wurde, sei es herbeigeführt durch Verstopfung der Spitzen, Fehlen von Flüssigkeit, Schaumbildung in der Flüssigkeit oder andere negative Einflüsse. Die Probe kann daraufhin für weitere Analysen markiert bzw. unter Umständen auch von einer weiteren Analyse ausgeschlossen werden. In der klinischen Diagnostik würde diese Information in vielen Fällen zu einem Reflex- bzw. Wiederholungstest führen, entweder auf dem gleichen System oder einer alternativen Methode.

Es zeigt sich dabei, dass durch die Entfernung von Zelldebris die Pipettierbarkeit (z.B. Aspiration der Lyseflüssigkeit) und Effizienz der automatisierten Aufreinigung verbessert wird, da es seltener zum Ausschluß einer Probe aufgrund eines schlechten TADM Profils kommt. Dadurch wird in der klinischen Diagnostik die Anzahl an Reflextests (erneute Durchführung des Tests, der zu einem Ergebnis führt) deutlich erniedrigt, was mit einer Kostenreduktion einhergeht.

### Beispiel 3 Aufreinignung von Nukleinsäuren aus Blutproben

Das Verfahren ist insbesondere auch zur Aufreinigung von Nukleinsäuren aus Blutproben geeignet, weil es eine verbesserte Entfernung von Hämoglobin oder Erythrozytenfragmenten aus dem Blut ermöglicht. Erfindungsgemäß werden unter nicht-chaotropen Bedingungen zunächst effizient Erythrozyten, Erythrozytenfragmente und auch freigesetztes Hämoglobin aus der Probe entfernt, welche spätere Verfahrensschritte stören können.

Gemäß einer ersten Variante wird zu einer Blutprobe (z.B. 100 µl EDTA-Gesamtblut) 400 µl Lysepuffer (z.B. 10 mMol TRIS-HCL, 0,1 mMol EDTA, 2% SDS, pH 8,0) gegeben. Anschließend erfolgt Zugabe von 50 µl Magnetpartikelsuspension (z.B. unbeschichtete oder silikabeschichtete Magnetpartikel) zu der Probe, Inkubation 10 min bei Raumtemperatur und Abtrennen der Partikel durch Anlegen eines Magnetfeldes. Aus der abgenommenen Probe können anschließend, wie oben beschrieben, unter chaotropen Bedingungen Nukleinsäuren isoliert werden.

Gemäß einer zweiten Variante wird die Blutprobe in einem hypotonen Lysepuffer (z.B. 25 mM TRIS HCl, pH 7,5, 10 mM KCl. 5 mM MgCl₂)aufgenommen, kurz inkubiert um die Erythrozyten zu lysieren und anschließend erfolgt die Zugabe Magnetpartikelsuspension (z.B. unbeschichtete oder silikabeschichtete Magnetpartikel) zu der Probe, Inkubation von 10 min bei Raumtemperatur und abtrennen der Partikel mitsamt der Erythorzytenfragmente und des Hämoglobins durch Anlegen eines Magnetfeldes. Anschließend erfolgt die Lyse der Leukozyten unter chaotropen Bedingungen, d.h. Freisetzung der Nukleinsäuren sowie Bindung dieser an frisch zugegebene Silika beschichtete Magnetpartikel. Proteinase K kann optional zuvor oder zeitgleich mit dem chaotropen Reagenz hinzugeführt werden.

### Beispiel 4: Herstellung von silika-beschichteten magnetischen Partikeln:

Die Herstellung von silika-beschichteten magnetischen Partikeln kann z.B. durch silika-Beschichtung von Magnetitpartikeln erfolgen. Bei den eingesetzten Magnetiten handelt es sich vorzugsweise um hydrophile, kommerziell erhältliche Eisenoxide (Fe₃0₄), die bevorzugt in enger Korngrössenverteilung und kugelförmiger Morphologie verfügbar sind. Magnetitpartikel sind kommerziell erhältlich, derartige Produkte werden beispielsweise von der Fa. Bayer AG unter dem Produktnamen BAYOXIDE E hergestellt. Geeignete Typen sind unter der Bezeichnung BAYOXIDE E8706, E8707, E8709 und E8710 erhältlich. Ähnliche Produkte werden auch von BASF unter dem Namen "Magnetic Pigment 340" bzw. "345" vertrieben. Obwohl mit allen genannten Produkten gute Ergebnisse erzielt werden können, wird vorzugsweise der Typ BAYOXIDE E 8707 oder E 8706 verwendet. Dieses magnetische Pigment besitzt eine kugelförmige Morphologie bei einem mittleren Teilchendurchmesser von 0,2 um und enger Korngrössenverteilung (ca. 0,1 bis 0,7 um). Als Ausgangsmaterialien zum Einführen von Silikatgruppen können sowohl Alkali- silikate (Natron- oder Kali-Wassergläser) als auch Kieselsole eingesetzt werden. Geeignete Wassergläser, die üblicherweise sehr hohe pH Werte (13-14) besitzen, werden von verschiedenen Firmen, beispielsweise Merck oder Cognis angeboten. Das zu beschichtende Material, beispielsweise Bayoxide E 8707, kann unter Rühren in eine verdünnte beispielsweise 1 % ige Wasserglaslösung gegeben werden. Nach einer Inkubation von ca. 30 Minuten wird abfiltriert, mit Wasser gewaschen und getrocknet. Gemäß einem exemplarischen Protokoll werden in 1000 ml einer wässrigen 0,25 % igen Wasserglaslösung (HK30; Cognis) unter Rühren 50 g Bayoxide E 8707 gegeben, anschließend wird noch 30 Min. bei RT nachgerührt Die Partikel werden abfiltriert, 5-mal mit Wasser und einmal mit Ethanol gewaschen und anschließend 5 Std. bei 80° C getrocknet.

## Patentansprüche

1. Automatisiertes System umfassend Mittel, die geeignet sind, ein Verfahren zum Aufreinigen von Nukleinsäuren aus einer biologischen Probe mit den folgenden Schritten durchzuführen:
a) Aufnehmen der Probe in einem ersten Probengefäß in einer wässrigen Lösung und Lysieren der Probe unter nicht-chaotropen Bedingungen;
b) Suspendieren von ersten magnetischen Partikeln in der Lösung,
c) Einbringen des ersten Probengefäßes in eine Probengefäßhalterung, wobei das Probengefäß in den Ringinnenraum eines mit der Probegefäßhalterung assoziierten Ringmagneten eingebracht wird;
d) Abtrennen der Lösung von den magnetischen Partikeln; und
e) Isolieren der Nukleinsäuren aus der Lösung,
wobei bei Einbringen des ersten Probengefäßes in den Ringinnenraum des mit der Probengefäßhalterung assoziierten Ringmagneten das jeweilige Probengefäß durch Bewegen in Richtung der Ringachse mindestens einmal aus dem Ringraum hinaus und wieder hinein bewegt wird,
wobei das System
- eine Ablage für mindestens ein Probengefäß;
- eine temperierbare Einrichtung mit mindestens einer Aufnahme für das Probengefäß;
- eine Probengefäßhalterung zur Aufnahme mindestens eines Probengefäßes;
- eine Einrichtung zum Transport der Probengefäßhalterung von der Ablage zu der temperierbaren Einrichtung;
- eine Einrichtung zum Transfer von Flüssigkeit aus einem Probengefäß und/oder in ein Probengefäß; und
- eine Steuerung zum Steuern der Transporteinrichtung, der Flüssigkeitstransfereinrichtung und zum Steuern der Temperatur der temperierbaren Einrichtung
aufweist
und die Probengefäßhalterung zur Aufnahme des mindestens einen Probengefäßes mindestens einen ringförmigen Magneten aufweist, in dessen Ringinnenraum das Probengefäß aufgenommen werden kann.

2. System nach Anspruch 1, **dadurch gekennzeichnet, dass** das erste Probengefäß mit einem hydrophoben Material aufgebaut ist und als hydrophobe Matrix fungiert.

3. System nach Anspruch 1, **dadurch gekennzeichnet, dass** die Probengefäßhalterung zur Aufnahme des mindestens einen Probengefäßes mindestens einen ringförmigen Magneten aufweist, dessen Ringinnendurchmesser an seiner engsten Stelle 4 bis 20 mm, bevorzugt 6 bis 12 mm, insbesondere 8 mm beträgt, wobei die Probengefäßhalterung bevorzugt zur Aufnahme eines Eppendorf-Reaktionsgefäßes dient und der Ringinnendurchmesser kleiner als der Durchmesser des Kragens des Eppendorf-Reaktionsgefäßes ist, jedoch größer als der Durchmesser des zylindrischen Bereichs unterhalb des Kragens.

4. System nach Anspruch 1, **dadurch gekennzeichnet, dass** der Ringinnenraum einen konischen Bereich aufweist, der sich der Form und Geometrie eines jeweiligen Probengefäßes anpasst.

5. System nach Anspruch 1, **dadurch gekennzeichnet, dass** es eine Probengefäßhalterung mit einer Mehrzahl von Aufnahmen für Probengefäße aufweist.

6. System nach Anspruch 1, **dadurch gekennzeichnet, dass** das Abtrennen der Lösung von den magnetischen Partikeln mittels einer Pipettenvorrichtung erreicht wird, deren einzelne Pipettierkanäle Drucksensoren enthalten.

7. System nach Anspruch 1, **dadurch gekennzeichnet, dass** die mittlere Größe der magnetischen Partikel weniger als 50 µm, bevorzugt weniger als 10 µm, besonders bevorzugt weniger als 0,5 µm beträgt.

8. System nach Anspruch 7, **dadurch gekennzeichnet, dass** die magnetischen Partikel einen Magnetitkern umfassen, der aus mindestens 90 Gewichtsprozent Fe₃O₄ besteht und dessen Oberfläche mit Silikat beschichtet ist.

9. System nach Anspruch 1, **dadurch gekennzeichnet, dass** die magnetischen Partikeln beim Abtrennen der Lösung an der vertikalen Wand des Probengefäßes abgelagert werden.

## Claims

1. An automated system comprising means that are suitable for carrying out a method for purifying nucleic acids from a biological sample, with the following steps:
a) collecting the sample in a first sample vessel in an aqueous solution and lysing the sample under nonchaotropic conditions;
b) suspending first magnetic particles in the solution;
c) placing the first sample vessel into a sample vessel holder, wherein the sample vessel is placed into the annular interior space of a ring magnet associated with the sample vessel holder;
d) separating the solution from the magnetic particles; and
e) isolating the nucleic acids from the solution,
wherein, when placing the first sample vessel into the annular interior space of the ring magnet associated with the sample vessel holder, the respective sample vessel is moved at least once out of the annular space and back in again by means of movement in the direction of the ring axis,
wherein the system has
- a rack for at least one sample vessel;
- a temperature-controllable device having at least one receptacle for the sample vessel;
- a sample vessel holder for receiving at least one sample vessel;
- a device for transporting the sample vessel holder from the rack to the temperature-controllable device;
- a device for transferring liquid from a sample vessel and/or into a sample vessel; and
- a control for controlling the transporting device, the liquid transferring device and for controlling the temperature of the temperature-controllable device;
and the sample vessel holder for receiving the at least one sample vessel has at least one annular magnet, in the annular interior space of which the sample vessel can be received.

2. The system as claimed in claim 1, wherein the first sample vessel is made of a hydrophobic material and acts as a hydrophobic matrix.

3. The system as claimed in claim 1, wherein the sample vessel holder for receiving the at least one sample vessel has at least one annular magnet, the ring inner diameter of which is, at its narrowest point, 4 to 20 mm, preferably 6 to 12 mm, in particular 8 mm, wherein the sample vessel holder preferably serves for receiving an Eppendorf reaction vessel and the ring inner diameter is less than the diameter of the collar of the Eppendorf reaction vessel, but greater than the diameter of the cylindrical region under the collar.

4. The system as claimed in claim 1, wherein the annular interior space has a conical region, which adapts itself to the shape and geometry of a respective sample vessel.

5. The system as claimed in claim 1, wherein it has a sample vessel holder with a plurality of receptacles for sample vessels.

6. The system as claimed in claim 1, wherein the separation of the solution from the magnetic particles is achieved by means of a pipette device, the individual pipetting channels of which contain pressure sensors.

7. The system as claimed in claim 1, wherein the average size of the magnetic particles is less than 50 pm, preferably less than 10 pm, particularly preferably less than 0.5 µm.

8. The system as claimed in claim 7, wherein the magnetic particles comprise a magnetite core, which consists of at least 90 percent by weight Fe₃O₄ and the surface of which is coated with silicate.

9. The system as claimed in claim 1, wherein, when separating the solution, the magnetic particles are deposited on the vertical wall of the sample vessel.

## Revendications

1. Système automatisé comprenant des moyens, qui conviennent pour réaliser un procédé de nettoyage d'acides nucléiques d'un échantillon biologique avec les étapes suivantes :
a) réception de l'échantillon dans un premier contenant d'échantillon dans une solution aqueuse et lyse de l'échantillon dans des conditions non chaotropiques ;
b) suspension de premières particules magnétiques dans la solution,
c) introduction du premier contenant d'échantillon dans un support de contenant d'échantillon, dans lequel le contenant d'échantillon est introduit dans l'espace intérieur annulaire d'un aimant annulaire associé au support de contenant d'échantillon ;
d) séparation de la solution des particules magnétiques ; et
e) isolation des acides nucléiques de la solution,
dans lequel lors de l'introduction du premier contenant d'échantillon dans l'espace intérieur annulaire de l'aimant annulaire associé au support de contenant d'échantillon, le contenant d'échantillon respectif est déplacé par déplacement dans la direction de l'axe annulaire au moins une fois hors de l'espace annulaire et à nouveau dedans,
dans lequel le système présente
- un dépôt pour au moins un contenant d'échantillon ;
- un dispositif tempérable avec au moins un logement pour le contenant d'échantillon ;
- un support de contenant d'échantillon pour la réception d'au moins un contenant d'échantillon ;
- un dispositif de transport du support de contenant d'échantillon du dépôt au dispositif tempérable ;
- un dispositif de transfert de liquide d'un contenant d'échantillon et/ou dans un contenant d'échantillon ; et
- une commande pour la commande du dispositif de transport, du dispositif de transfert de liquide et pour la commande de la température du dispositif tempérable
et le support de contenant d'échantillon pour la réception de l'au moins un contenant d'échantillon présente au moins un aimant annulaire, dans lequel espace intérieur annulaire le contenant d'échantillon peut être reçu.

2. Système selon la revendication 1, **caractérisé en ce que** le premier contenant d'échantillon est construit avec un matériau hydrophobe et fonctionne comme matrice hydrophobe.

3. Système selon la revendication 1, **caractérisé en ce que** le support de contenant d'échantillon pour la réception de l'au moins un contenant d'échantillon présente au moins un aimant annulaire, dont le diamètre intérieur annulaire est à son point le plus étroit de 4 à 20 mm, de préférence de 6 à 12 mm, en particulier de 8 mm, dans lequel le support de contenant d'échantillon sert de préférence à la réception d'un tube Eppendorf et le diamètre intérieur annulaire est inférieur au diamètre du col du tube Eppendorf, cependant supérieur au diamètre de la zone cylindrique sous le col.

4. Système selon la revendication 1, **caractérisé en ce que** l'espace intérieur annulaire présente une zone conique, qui s'adapte à la forme et à la géométrie d'un contenant d'échantillon respectif.

5. Système selon la revendication 1, **caractérisé en ce qu'**il présente un support de contenant d'échantillon avec une pluralité de logements pour contenants d'échantillon.

6. Système selon la revendication 1, **caractérisé en ce que** la séparation de la solution des particules magnétiques est obtenue au moyen d'un dispositif de pipette, dont les différents canaux de pipetage contiennent des capteurs de pression.

7. Système selon la revendication 1, **caractérisé en ce que** la taille moyenne des particules magnétiques est inférieure à 50 µm, de préférence inférieure à 10 µm, de manière particulièrement préférée inférieure à 0,5 µm.

8. Système selon la revendication 7, **caractérisé en ce que** les particules magnétiques comprennent un noyau de magnétite, qui se compose d'au moins 90 pour cent en poids de Fe₃O₄ et dont la surface est revêtue de silicate.

9. Système selon la revendication 1, **caractérisé en ce que** les particules magnétiques lors de la séparation de la solution sont déposées sur la paroi verticale du contenant d'échantillon.
